# EUROPEAN PATENT APPLICATION

(11) **EP 4 098 269 A1**
(43) Date of publication of application: **07.12.2022**
(21) Application number: 21747308.1
(22) Date of filing: 20.01.2021
(51) Int. Cl.: A61K 35/39, A61K 9/48, A61K 47/12, A61K 47/36, A61K 47/42, A61P 3/10

(54) **METHOD FOR PRODUCING PANCREATIC ISLET-CONTAINING CAPSULE**

(30) Priority: 28.01.2020 JP 2020011625
(71) Applicant: Otsuka Pharmaceutical Factory, Inc., Naruto-shi, Tokushima 772-8601 (JP)
(72) Inventor: AZUMA, Koji, Naruto-shi, Tokushima 772-8601 (JP); NISHIMURA, Masuhiro, Naruto-shi, Tokushima 772-8601 (JP); IIZUKA, Naho, Naruto-shi, Tokushima 772-8601 (JP); TAMURA, Hirofumi, Naruto-shi, Tokushima 772-8601 (JP)
(74) Representative: Vossius & Partner Patentanwälte Rechtsanwälte mbB
(86) International application number: PCT/JP2021/001767
(87) International publication number: WO 2021/153365

(57) **Abstract**

The present invention provides an improved method for producing pancreatic islet-containing capsules.

A method for producing pancreatic islet-containing capsules, comprising the steps of:
(a) preparing a sodium alginate solution A containing pancreatic islets at a concentration of 10,000 IEQ/mL or more;
(b) adding the sodium alginate solution to a divalent cation solution dropwise, and collecting gelled particles;
(c) adding the particles collected in step (b) to a poly-L-ornithine solution A, followed by stirring and then collecting particles;
(d) adding the particles collected in step (c) to a sodium alginate solution B, followed by stirring and then collecting particles; and
(e) adding the particles collected in step (d) to a sodium citrate solution, followed by stirring and then collecting particles.

## Description

### Technical Field

Technologies relating to encapsulated pancreatic islets are disclosed.

### Background Art

Pancreatic islet transplantation is a means of treatment for diabetes with a relatively low burden on the human body; however, strong immunosuppression may be sometimes required for long-term survival of transplanted pancreatic islets. A method for transplanting a large number of micro-encapsulated pancreatic islets into an abdominal cavity has been discussed. This method does not require immunosuppression; however, sufficient therapeutic results have not yet been obtained.

### Citation List

### Patent Literature

PTL 1: WO2001/052871

### Non-patent Literature

NPL 1: Methods in Enzymology, Vol. 137, 575-580, 1988
NPL 2: Transplantation, Vol. 53, 1180-1183, No. 6, June 1992
NPL 3: Bioartificial Pancreas, Vol. 98, No. 6, 1996, 1417-1422
NPL 4: Drug Delivery System, Vol. 12, No. 2, 1997
NPL 5: Journal of Biomedical Materials Research Part B: Applied Biomaterials, 2013, Vol. 101B, Issue 2, 258-268

### Summary of Invention

### Technical Problem

An object is to provide an improved method for producing pancreatic islet-containing capsules.

### Solution to Problem

As a means for achieving the above object, the following invention is provided.
Item 1. A method for producing pancreatic islet-containing capsules, comprising the steps of:
   (a) preparing a sodium alginate solution A containing pancreatic islets at a concentration of 10,000 IEQ/mL or more;
   (b) adding the sodium alginate solution to a divalent cation solution dropwise, and collecting gelled particles;
   (c) adding the particles collected in step (b) to a poly-L-ornithine solution A, followed by stirring and then collecting particles;
   (d) adding the particles collected in step (c) to a sodium alginate solution B, followed by stirring and then collecting particles; and
   (e) adding the particles collected in step (d) to a sodium citrate solution, followed by stirring and then collecting particles.
Item 2. The method according to Item 1, wherein the pancreatic islets are obtained from a one- to three-week-old neonatal pig.
Item 3. The method according to Item 1 or 2, wherein the pancreatic islet-containing capsules have an average diameter of 550 to 750 µm.
Item 4. The method according to any one of Items 1 to 3, wherein the sodium alginate solution A has a sodium alginate concentration of 1 w/v% or more to 3 w/v% or less.
Item 5. The method according to any of Items 1 to 4, wherein the poly-L-ornithine solution A has a poly-L-ornithine concentration of 0.05 w/v% or more to 3 w/v% or less.
Item 6. The method according to any one of Items 1 to 5, wherein the sodium alginate solution B has a sodium alginate concentration of 0.1 w/v% or more to 0.3 w/v% or less.
Item 7. The method according to any of Items 1 to 6, wherein in step (d), the particles collected in step (c) are added to the sodium alginate solution B after being added to the poly-L-ornithine solution B, stirred, and then collected.
Item 8. The method according to Item 7, wherein the poly-L-ornithine solution B has a poly L-ornithine concentration of 0.01 w/v% or more to less than 0.1 w/v%.

### Advantageous Effects of Invention

The present invention provides encapsulated pancreatic islets having improved glucose responsiveness.

### Brief Description of Drawings

Fig. 1 shows micrographs of pancreatic islets immediately after encapsulation, and pancreatic islets cultured for 25 days after encapsulation. a to d indicate immediately after encapsulation, and e to h indicate after 25 days of culture. a and e are produced using 8,000 IEQ/mL pancreatic islets, b and f are produced using 12,000 IEQ/mL pancreatic islets, c and g are produced using 16,000 IEQ/mL pancreatic islets, and d and h are produced using 20,000 IEQ/mL pancreatic islets.
Fig. 2 shows the measurement results of glucose responsiveness of pancreatic islets cultured for 25 days after encapsulation.
Fig. 3 shows the measured glucose responsiveness of pancreatic islets cultured for 25 days after encapsulation as a stimulation index (SI).
Fig. 4 shows the measurement results of glucose responsiveness of pancreatic islet-containing capsules with different sizes.
Fig. 5 shows the measurement results of SI of pancreatic islet-containing capsules with different sizes.

### Description of Embodiments

The method for producing pancreatic islet-containing capsules preferably includes the following steps (a) to (e):
(a) preparing a sodium alginate solution A containing pancreatic islets at a concentration of 10,000 IEQ/mL or more;
(b) adding the sodium alginate solution dropwise to a divalent cation solution, and collecting gelled particles;
(c) adding the particles collected in step (b) to a poly-L-ornithine solution A, followed by stirring and then collecting particles;
(d) adding the particles collected in step (c) to a sodium alginate solution B, followed by stirring and then collecting particles; and
(e) adding the particles collected in step (d) to a sodium citrate solution, followed by stirring and then collecting particles.

The pancreatic islets used in step (a) preferably include insulin-producing β-cells, glucagon-containing α-cells, somatostatin-secreting delta cells, and pancreatic polypeptide-containing cells (PP cells). Most of the pancreatic islets are preferably insulin-producing β-cells.

The source of pancreatic islets is not particularly limited; the pancreatic islets are preferably derived from a human, a pig, a mouse, a rat, a monkey, or a dog. In one embodiment, the pancreatic islets are preferably derived from a pig, and pancreatic islets derived from a neonatal pig (e.g., 3-day-old to 4-week-old, or 7-day-old to 3-week-old) are preferred. Pancreatic islets can be obtained by employing any method known in this technical field. For example, a method using Liberase as a digestive enzyme (T.J. Cavanagh et al., Transplantation Proceedings, 30, 367 (1998)) can be preferably used.

The size of the pancreatic islet is preferably 50 µm or more to 400 µm or less. The size of the pancreatic islet can be measured using a microscopic micrometer. The pancreatic islets preferably contain β-cells at a concentration of 10% or more. The upper limit of the ratio of the β-cells is not particularly limited; it is, for example, 80%.

The physical properties of the sodium alginate constituting the sodium alginate solution A are not particularly limited.

The sodium alginate concentration of the sodium alginate solution A is not particularly limited. For example, it is preferably 1 w/v% or more to 3 w/v% or less.

It is preferable that the sodium alginate solution A contain pancreatic islets at a concentration of 10,000 IEQ/mL or more, from the viewpoint of improving the glucose responsiveness of pancreatic islets after encapsulation. IEQ means the number of standard pancreatic islets having a diameter of 150 µm. The concentration of pancreatic islets is preferably 11,000 IEQ/mL or more, or 12,000 IEQ/mL or more. The upper limit of the concentration of pancreatic islets is not particularly limited. For example, it is 30,000 IEQ/mL or less, or 25,000 IEQ/mL or less.

The specific method for preparing the sodium alginate solution A is not limited as long as the sodium alginate solution A is prepared so that pancreatic islets are contained at a concentration mentioned above. For example, the sodium alginate solution A can be obtained by dissolving an appropriate amount of sodium alginate in a saline solution, adding an appropriate amount of pancreatic islets thereto, and optionally performing stirring.

The divalent cation solution used in step (b) is not limited, as long as gelled particles (capsules) encapsulating pancreatic islets can be obtained by adding the sodium alginate solution A dropwise to the divalent cation solution. Examples of divalent cations constituting the divalent cation solution include salts that release divalent metal ions in an aqueous solution (e.g., calcium chloride, calcium lactate, barium chloride, and strontium chloride). In one embodiment, a preferred salt is calcium chloride, and a preferred divalent cation solution is a calcium chloride solution.

The concentration of divalent cations in the divalent cation solution is not particularly limited. For example, it can be set in the range of 50 to 500 mM.

The style of dropwise addition of the sodium alginate solution A to the divalent cation solution is not limited as long as gelled particles encapsulating pancreatic islets can be obtained. In one embodiment, the sodium alginate solution A is preferably added dropwise to the divalent cation solution through an appropriately sized needle.

The gelled particles can be collected by any method. For example, the gelled particles can be collected by allowing a divalent cation solution in which the gelled particles are formed to stand for a certain period of time, and removing the supernatant; or by repeating such a step.

The poly-L-ornithine solution A used in step (c) is not particularly limited as long as it is capable of coating the gelled particles formed in step (b). For example, the poly-L-ornithine solution A can be prepared by dissolving poly-L-ornithine in a physiological saline solution.

The concentration of poly-L-ornithine in the poly-L-ornithine solution A is not particularly limited; it can be adjusted, for example, to 0.05 w/v% or more to 3.0 w/v% or less. In one embodiment, the concentration of poly-L-ornithine in the poly-L-ornithine solution A is preferably 0.1 w/v% or more to 2.0 w/v% or less.

Step (c) of "adding the particles collected in step (b) to a poly-L-ornithine solution A" also includes an embodiment in which the poly-L-ornithine solution A is added to the gelled particles collected in step (b). The stirring speed and time in step (c) is not limited. For example, stirring is preferably performed at 20 to 300 rpm for 1 to 20 minutes.

The gelled particles can be collected in any manner in step (c). For example, the gelled particles can be collected by allowing the poly-L-ornithine solution A to stand for a certain period of time, and removing the supernatant. The collected gelled particles preferably have a structure in which the surface of a gelled layer due to alginic acid is coated with poly-L-ornithine.

The concentration of the sodium alginate solution B used in step (d) may be the same or different from that of the sodium alginate solution A used in step (a). In one embodiment, the sodium alginate concentration of the sodium alginate solution B is preferably lower than the sodium alginate concentration of the sodium alginate solution A in terms of ease of coating operation. For example, when the sodium alginate concentration of the sodium alginate solution A is 1 w/v% or more to 3 w/v% or less, the sodium alginate concentration of the sodium alginate solution B is preferably 0.1 w/v% or more to 0.3 w/v% or less.

Step (d) of "adding the particles collected in step (c) to a sodium alginate solution B" also includes an embodiment in which the sodium alginate solution B is added to the gelled particles collected in step (c). The stirring speed and time in step (d) is not limited. For example, stirring is preferably performed for 1 to 15 minutes at 20 to 300 rpm.

The gelled particles can be collected in any manner in step (d). For example, the gelled particles can be collected by allowing the sodium alginate solution B to stand for a certain period of time, and removing the supernatant. The collected gelled particles preferably have a structure in which the surface of a gelled layer due to alginic acid is coated with poly-L-ornithine, and a gelled layer due to alginic acid is further formed thereon.

In one embodiment, it is preferable that the particles collected in step (c) be added to the poly L-ornithine solution B, stirred, and then collected before being added to the sodium alginate solution B; and then further added to the sodium alginate solution B in step (d). The poly-L-ornithine solution B may have a concentration that is the same as or different from that of the poly-L-ornithine solution A. In one embodiment, the poly-L-ornithine concentration of the poly-L-ornithine solution B is preferably lower than the poly-L-ornithine concentration of the poly-L-ornithine solution A. For example, the poly-L-ornithine concentration of the poly-L-ornithine solution B is preferably 0.01 w/v% or more to less than 0.1 w/v%.

The sodium citrate solution used in step (e) preferably contains sodium citrate at a concentration of 0.5 to 3 w/v%. The sodium citrate solution is added for the purpose of stimulating insulin release by chelating cations in the gelled particles to put the gel close to a liquid state.

Step (e) of "adding the particles collected in step (d) to a sodium citrate solution" also includes an embodiment in which the sodium citrate solution is added to the gelled particles collected in step (d). The stirring speed and time in step (e) is not limited. For example, stirring is preferably performed for 1 to 15 minutes at 20 to 300 rpm.

The gelled particles can be collected in any manner in step (e). For example, the gelled particles can be collected by allowing the sodium citrate solution to stand for a certain period of time, and removing the supernatant. The collected gelled particles are considered to contain an inner content that is more of a liquid state than the outer content.

The thus-obtained pancreatic islet-containing capsules have excellent glucose responsiveness, and stably maintain excellent glucose responsiveness. In one embodiment, the gelled particles (pancreatic islet-containing capsules) collected in step (e) preferably have an average particle diameter of 2000 µm or less, more preferably 1500 µm or less, or 1000 µm or less, from the viewpoint of exhibiting high glucose responsiveness. The lower limit of the size of the pancreatic islet-containing capsule is not particularly limited; it is, for example, 100 µm or more, 200 µm or more, 300 µm or more, 400 µm or more, or 500 µm or more. The average diameter is the value obtained by measuring the sizes of 20 to 50 pancreatic islet-containing capsules with a microscopic micrometer, and calculating the average thereof.

### Examples

The present invention is described in more detail by means of Examples below; however, the present invention is not limited to these Examples.

### 1. Preparation of encapsulated pancreatic islets

Neonatal pig-derived pancreatic islets isolated from a 14-day-old pig were suspended in a 1.7 w/v% sodium alginate solution (PRONOVA) at a concentration of 8,000 IEQ/mL, 12,000 IEQ/mL, 16,000 IEQ/mL, or 20,000 IEQ/mL. Each resultant was passed through a needle, and added dropwise to a 109 mM calcium chloride solution while cutting the suspension by airflow. Solidified alginate beads were collected from the calcium chloride solution, and added to a 0.075 w/v% poly-L-ornithine (PLO) (molecular weight 5,000 to 15,000) solution, followed by stirring for 10 minutes. The beads were collected, and further added to a 0.038 w/v% PLO solution, followed by stirring for 6 minutes and collecting. The beads were then added to a 0.17 w/v% sodium alginate solution, followed by stirring for 6 minutes. Finally, the beads were added to a 1.6% sodium citrate solution, followed by stirring for 2 minutes, thus obtaining encapsulated pancreatic islets. The resulting encapsulated pancreatic islets were cultured in a CO₂ incubator at 37°C for 25 days.

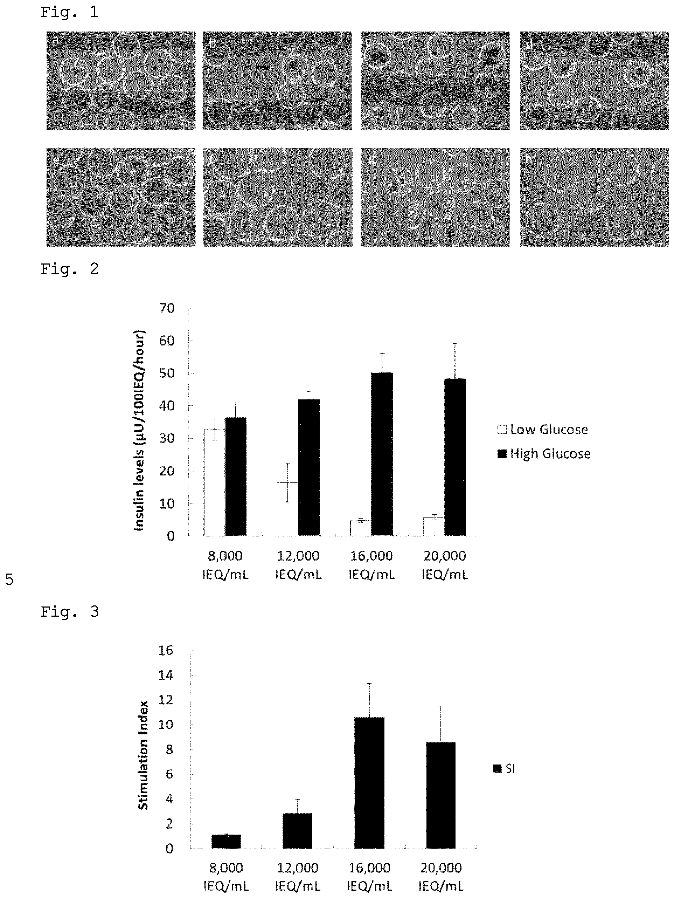

Immediately after encapsulation and after 25 days of culture, a small amount of the encapsulated pancreatic islets was taken from a flask, and transferred to a 35-mm dish. The dish was observed using an inverted microscope (4x), and the encapsulated pancreatic islets were photographed (Fig. 1). In Fig. 1, a to d indicate immediately after encapsulation, while e to h indicate after 25 days of culture, a and e were produced using 8,000 IEQ/mL pancreatic islets, b and f were produced using 12,000 IEQ/mL pancreatic islets, c and g were produced using 16,000 IEQ/mL pancreatic islets, and d and h were produced using 20,000 IEQ/mL pancreatic islets. The capsule diameter was about 600 µm on average.

### 2. Evaluation of glucose responsiveness

500IEQ of each of the encapsulated pancreatic islets that had been cultured for 25 days after encapsulation in an RPMI 1640 medium supplemented with 2% pig serum, 10 mM nicotinamide, and antibiotics was introduced into a Netwell insert with a 74 µm mesh size (produced by Corning). After washing, the inserts containing pancreatic islets were allowed to stand in a 500 mg/L low-glucose medium for 1 hour, and then the medium was collected. Next, the inserts containing encapsulated pancreatic islets were allowed to stand in a 3750 mg/L high-glucose medium for 1 hour, and then the medium was collected. The insulin concentrations in the collected media were measured using an ELISA method (Fig. 2). The ratio of the insulin concentration in a high-glucose medium to the insulin concentration in a low-glucose medium was calculated as a stimulation index (SI) (Fig. 3). As shown in Fig. 2, it was found that capsules prepared using pancreatic islets with a concentration higher than 8,000 IEQ/mL showed high glucose responsiveness, as compared to capsules prepared using 8,000 IEQ/mL pancreatic islets.

### 3. Capsule size and glucose responsiveness

In the same procedure as in Item 1 above, encapsulated pancreatic islets having a diameter of about 500 to 1800 µm were produced by adjusting the airflow speed. 3000-µm encapsulated pancreatic islets were produced by connecting an outer cylinder of 18G indwelling needle to a syringe, and adding the suspension dropwise. Pancreatic islet-containing capsules were produced using a suspension containing 16,000 IEQ/mL neonatal pig-derived pancreatic islets.

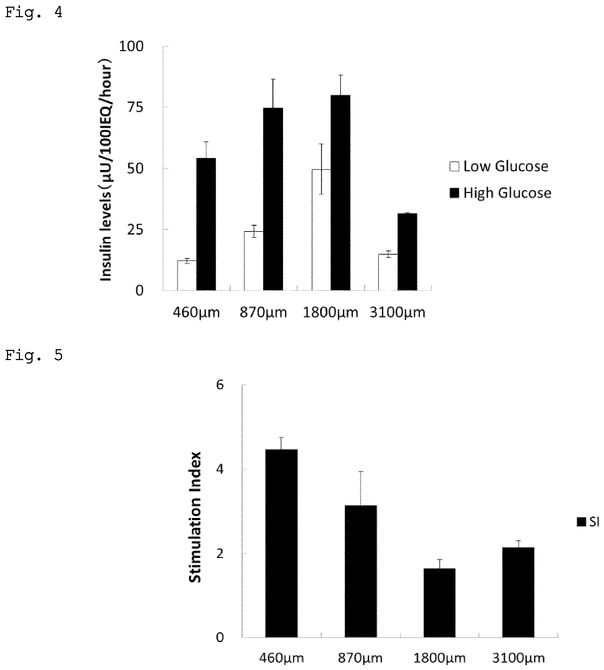

Figs. 4 and 5 show the results of the glucose responsiveness of the resulting pancreatic islet-containing capsules, which were evaluated in the same manner as in Item 2 above. The results indicated that capsules having a diameter of 2000 µm or less, 1800 µm or less, or 1500 µm or less had preferable glucose responsiveness.

## Claims

1. A method for producing pancreatic islet-containing capsules, comprising the steps of:
(a) preparing a sodium alginate solution A containing pancreatic islets at a concentration of 10,000 IEQ/mL or more;
(b) adding the sodium alginate solution to a divalent cation solution dropwise, and collecting gelled particles;
(c) adding the particles collected in step (b) to a poly-L-ornithine solution A, followed by stirring and then collecting particles;
(d) adding the particles collected in step (c) to a sodium alginate solution B, followed by stirring and then collecting particles; and
(e) adding the particles collected in step (d) to a sodium citrate solution, followed by stirring and then collecting particles.

2. The method according to claim 1, wherein the pancreatic islets are obtained from a one- to three-week-old neonatal pig.

3. The method according to claim 1 or 2, wherein the pancreatic islet-containing capsules have an average diameter of 550 to 750 pm.

4. The method according to any one of claims 1 to 3, wherein the sodium alginate solution A has a sodium alginate concentration of 1 w/v% or more to 3 w/v% or less.

5. The method according to any of claims 1 to 4, wherein the poly-L-ornithine solution A has a poly-L-ornithine concentration of 0.05 w/v% or more to 3 w/v% or less.

6. The method according to any one of claims 1 to 5, wherein the sodium alginate solution B has a sodium alginate concentration of 0.1 w/v% or more to 0.3 w/v% or less.

7. The method according to any of claims 1 to 6, wherein in step (d), the particles collected in step (c) are added to the sodium alginate solution B after being added to the poly-L-ornithine solution B, stirred, and then collected.

8. The method according to claim 7, wherein the poly-L-ornithine solution B has a poly L-ornithine concentration of 0.01 w/v% or
more to less than 0.1 w/v%.
